# EUROPEAN PATENT APPLICATION

(11) **EP 4 725 529 A1**
(43) Date of publication of application: **15.04.2026**
(21) Application number: 25207617.9
(22) Date of filing: 08.10.2025
(51) Int. Cl.: A61M 16/04, A61M 16/08

(54) **ORAL POSITIVE EXPIRATORY PRESSURE DEVICE**

(30) Priority: 09.10.2024 US 202463705490 P
(71) Applicant: PEP Buddy LLC, Wyoming, OH 45215 (US)
(72) Inventor: Zafar, Muhammad Ahsan, Mason, OH, 45040 (US); Panos, Ralph J., Wyoming, OH, 45215 (US)
(74) Representative: Mzb PartmbB

(57) **Abstract**

A compact, hands-free, oral positive expiratory pressure (PEP) device designed to optimize respiratory mechanics by providing controlled resistance during exhalation. The device slows the respiratory rate, prolongs exhalation, generates positive airway pressure, and thereby reduces breathlessness, enhances oxygenation, and expands functional lung capacity. It is particularly beneficial in conditions such as chronic obstructive pulmonary disease (COPD), tracheobronchomalacia, excessive dynamic airway collapse (EDAC), bronchiectasis, atelectasis, and acute respiratory distress syndromes, while also serving broader functions like stress reduction, cardiovascular support, and respiratory muscle training. The device is lightweight, portable, and lanyard-supported, allowing continuous availability for daily use and physical activity. Structurally, it is conical, features a primary airflow chamber, a mouth opening, and one or more resistance orifices that regulate exhalation pressure. By maintaining consistent PEP levels (ranging approximately 4-15 cm H₂O), the device helps prevent airway collapse, promotes alveolar recruitment, and improves the ventilation-perfusion ratio.

## Description

### CROSS REFERENCE TO RELATED APPLICATION

The present application claims priority to US provisional patent application number 63/705,490 filed on October 9, 2024, the disclosure of which is incorporated by reference herein in its entirety.

### TECHNICAL FIELD

The present invention concerns a portable, hands-free oral device that produces positive expiratory pressure for therapeutic and wellness purposes.

### BACKGROUND

Respiratory disorders involving obstructed or unstable airways pose significant health challenges. In COPD, emphysema, and bronchiectasis, small airway obstruction leads to air trapping, dyspnea, and reduced exercise tolerance. Similarly, central airway collapse occurs in conditions such as EDAC and tracheobronchomalacia, producing severe shortness of breath and impaired quality of life.

Dynamic hyperinflation-caused by shortened exhalation during exertion-is a key mechanism for exercise intolerance in COPD. It limits expiratory airflow, worsens gas exchange, reduces oxygen levels, and stresses the cardiovascular system. Current interventions, such as pursed-lip breathing, aim to counter these effects by generating positive pressure during exhalation. However, such self-administered techniques are inconsistent and difficult to sustain.

Conventional PEP devices, such as oscillatory valves (e.g., Acapella, Aerobika, Flutter), are primarily hand-held, multi-component, or require external power or airflow. While they offer some therapeutic benefits, they are inconvenient during physical activity or daily life. Similarly, incentive spirometry, a mainstay for prevention of postoperative atelectasis, relies on inspiratory maneuvers that are often painful, poorly tolerated, and lack consistent efficacy.

Thus, there is a clear gap in respiratory care for a simple, non-invasive, portable, and hands-free device that can deliver consistent positive expiratory pressure while supporting multiple therapeutic and preventive applications.

### SUMMARY

Atelectasis and other perioperative pulmonary complications affect up to 90% of patients after surgery. Traditional lung expansion techniques often fail due to poor adherence or mechanical complexity. Moreover, individuals with chronic respiratory conditions require interventions that can be incorporated seamlessly into daily life rather than restricted to hospital or clinic settings.

The invention addresses these unmet needs by providing:
- A lightweight, hands-free device that ensures adherence and ease of use.
- Consistent positive expiratory pressure to stent open airways and prevent collapse.
- Lung expansion through alveolar recruitment at pressures between 4-15 cm H₂O.
- Support for relaxation, stress reduction, and sleep regulation through breathing pattern modification.
- Cardiovascular benefits via parasympathetic stimulation, blood pressure reduction, and improved venous return.
- Respiratory muscle conditioning by applying a mild resistive load during exhalation.
In contrast to existing bulky or complex systems, PEP Buddy combines clinical utility with user convenience, making it suitable for both medical and lifestyle applications.

Existing devices like Acapella, Aerobika, EZPAP, and Flutter Valve share a common limitation-they are relatively bulky, require manual handling, or involve multiple components. Many are not suitable for active use or for integration into daily activities. Some require external power or airflow, which further reduces practicality.

PEP Buddy distinguishes itself through:
- Hands-free usability with a neck lanyard attachment.
- Minimalist, single-piece conical design with airflow resistance orifices.
- Portability and convenience for use during walking, exercise, or normal daily activity.
- Wider clinical and non-clinical applications (e.g., lung disease management, prevention of atelectasis, stress relief, mindfulness, cardiovascular support, insomnia treatment).
Capability to deliver specific expiratory pressures in the therapeutic range (4-15 cm H₂O) not consistently achieved by pursed-lip breathing or comparable devices.

### BRIEF DESCRIPTION OF THE DRAWINGS

The objectives and features of the invention will become more readily apparent from the following detailed description taken in conjunction with the accompanying drawings in which:

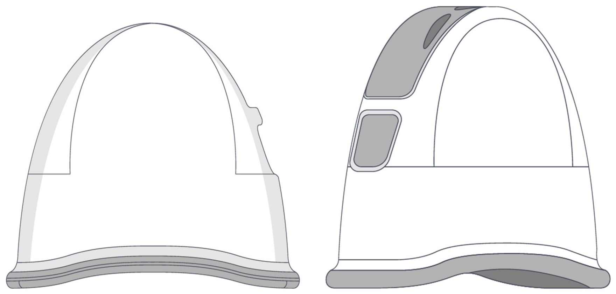

FIGURE 1 illustrates an external perspective view of the PEP Buddy device in its fully assembled form. The figure shows the proximal (base) mouthpiece portion designed to be held securely within the user's lips and the distal tapered section incorporating one or more resistance-generating orifices. The overall structure demonstrates the compact, lightweight, and hands-free design that differentiates the device from conventional incentive spirometry or bulky positive airway pressure systems.

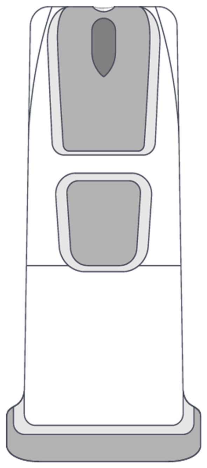

FIGURE 2 presents a side external view of the device depicting its small size and minimalistic design. The horizontal bar provides a site for attaching a lanyard to hang the device around the neck or on the writs for easy and quick access.

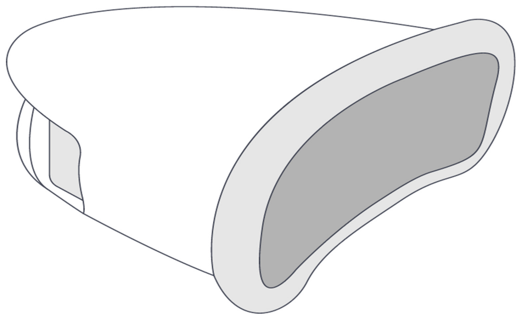

FIGURE 3 presents the view of the PEP Buddy device from the base-side where the mouthpiece is present, depicting the internal hollow conical chamber and airflow pathway. This view clarifies how the device's geometry and orifice structure regulate airflow resistance to create positive expiratory pressure (PEP). The illustration highlights the functional simplicity of the design, which requires no external power source or complex assembly.

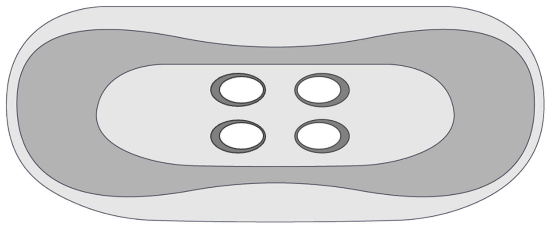

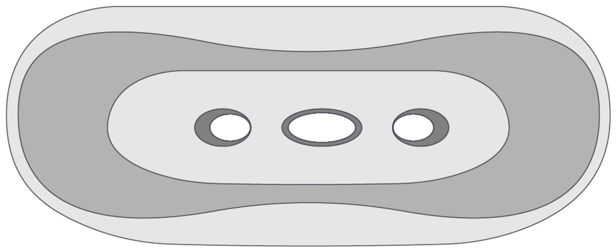

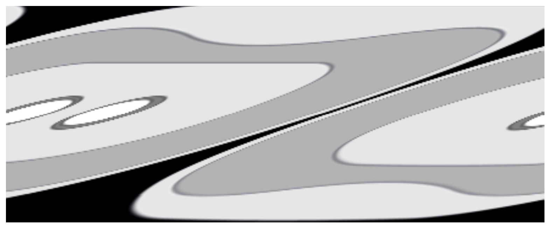

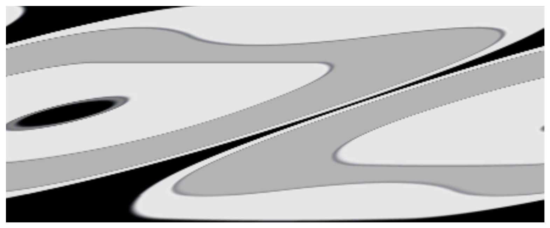

FIGURE 4 provides a top view of the device at the tapered (conical) end. This end has small holes (orifices) that can be of different numbers and/ or sizes. The small orifices provide airflow resistance when the user breathes out from the mouthpiece. This airflow resistance creates the back pressure or positive expiratory pressure (PEP). The device shown in Figure 4A has 4-holes and generates less PEP. Figure 4B, C and D show the variants of PEP buddy with 3-holes, 2-holes, and single hole. The fewer the number of holes, the higher the airflow resistance and PEP generation.

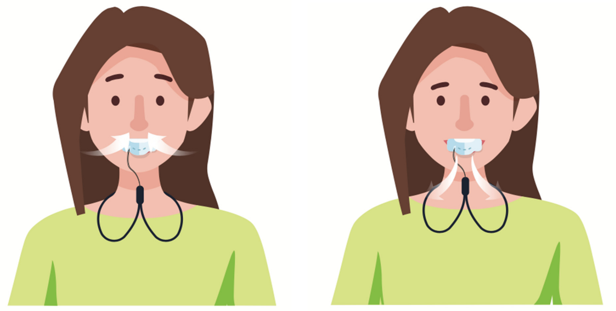

FIGURE 5 depicts the device in active use by a subject. The illustration shows the proximal end held in the oral cavity with a tight lip seal, while exhalation is directed through the distal resistance orifice. This figure emphasizes the hands-free capability of the device, enhancing usability in ambulatory settings, during exercise, or in patients with limited hand mobility.

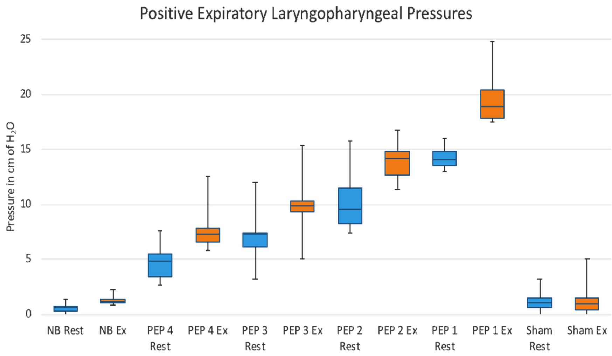

FIGURE 6 illustrates the positive expiratory pressures generated using different grades of oral PEP devices in normal subjects at rest and during exercise. The data confirm the ability of the device to deliver clinically relevant and reproducible expiratory pressures under varying physiologic conditions. Adjustments to the number of holes (orifices) on the distal (conical) end lead to different levels of PEP. The more or larger the holes, the less is the PEP. This figure highlights the adaptability of the device to diverse clinical contexts, enabling use in both healthy individuals for respiratory training and in patients with conditions such as atelectasis, COPD, or ARDS requiring tailored pressures.

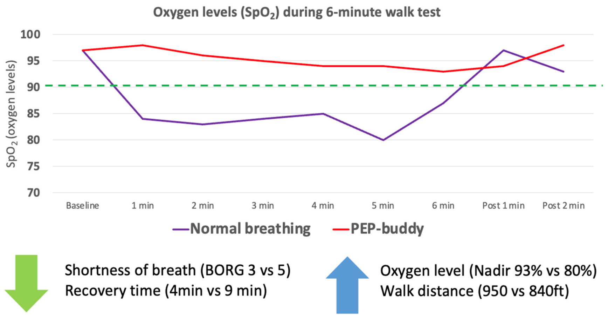

FIGURE 7: is a graph illustrating the effect of the device on exertional oxygen saturation in a subject with COPD and exertional desaturation. The results show improved lung expansion (lower lung ultrasound scores) and enhanced oxygenation (SpO₂ levels). This figure supports the utility of the device in postoperative care, atelectasis prevention, and treatment of acute respiratory failures including ARDS and pneumonia.

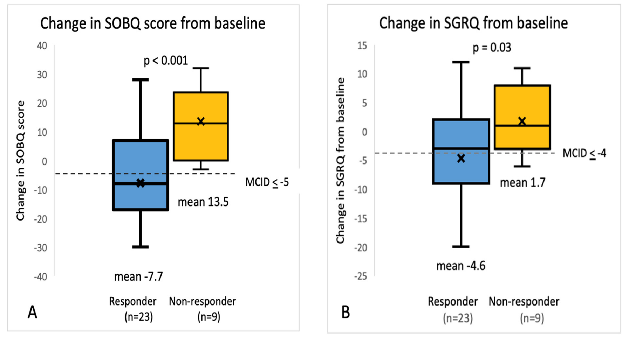

FIGURE 8: displays clinical outcomes of device use in COPD patients, measured through validated instruments. Improvements are shown in the Shortness of Breath Questionnaire (SOBQ) and the St. George's Respiratory Questionnaire (SGRQ), with differences exceeding the minimal clinically important difference (MCID). These results demonstrate the device's efficacy in reducing dyspnea and improving quality of life.

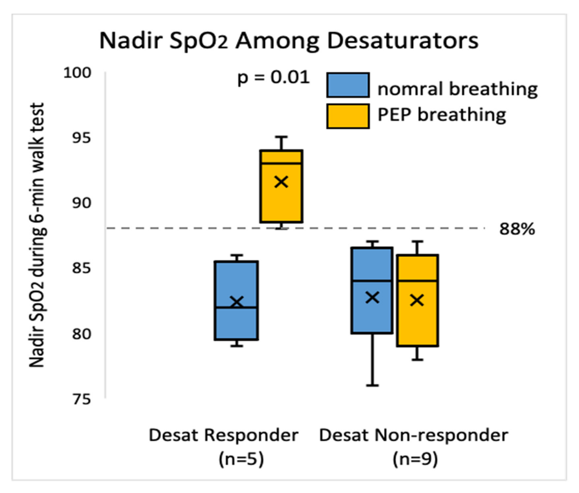

FIGURE 9 illustrates the effect of the device on exertional desaturation in COPD responders, showing a significant reduction in oxygen desaturation events during exertion. These findings validate the ability of the PEP Buddy to enhance exercise tolerance and functional capacity.

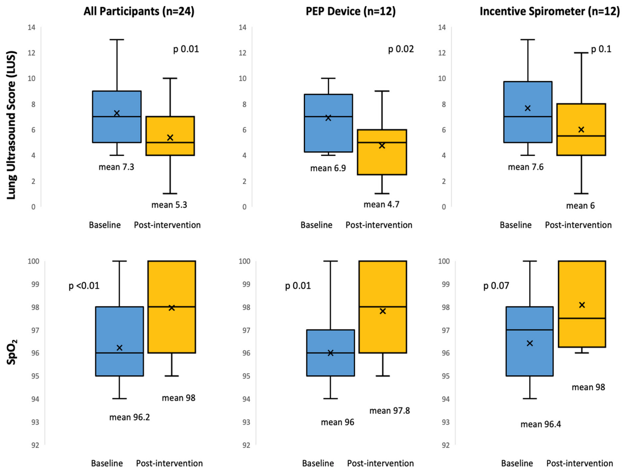

FIGURE 10 compares the effect of PEP Buddy with incentive spirometry in postoperative patients with atelectasis. Lung ultrasound scores (LUS) show greater reduction in patients using PEP Buddy, indicating superior lung aeration and expansion. Additionally, SpO₂ improvements confirm enhanced oxygenation, reinforcing the device's role as a more effective and user-friendly alternative to incentive spirometry.

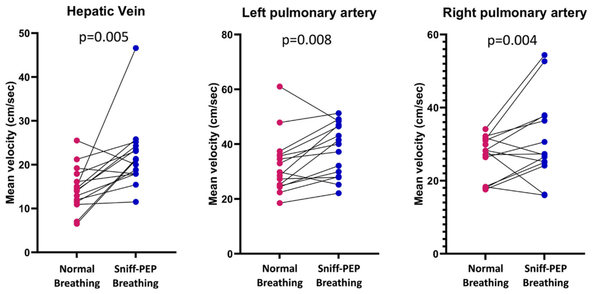

FIGURE 11 shows the changes in central venous blood flow circulation with the use of PEP breathing and augmented inhalation (Sniff-PEP) compared to normal breathing. Such breathing creates augmented shifts in intra-thoracic pressures with positive expiratory pressure (PEP) and a greater negative intra-thoracic pressure during inhalation, causing an increase in central blood flow. The increase in blood flow is depicted in hepatic vein, left pulmonary artery, and right pulmonary artery in patients with single heart ventricle and Fontan circulation. The same principle can be utilized in other conditions that lead to heart failure or reduced cardiac function.

### DETAILED DESCRIPTION OF PARTICULAR EMBODIMENTS

### Additional Indications and Uses for Positive Expiratory Pressure Device

While the positive expiratory pressure (PEP) device has well-documented use in the management of obstructive lung diseases, its applications extend far beyond pulmonary rehabilitation. Controlled breathing through regulated expiratory resistance not only stabilizes airways but also reshapes respiratory patterns in ways that influence cardiovascular, neurological, and psychological systems.

The device may therefore be employed during mindfulness practices, yoga, meditation, pregnancy-related relaxation, and stress-reduction exercises, providing individuals with a reliable tool to regulate exhalation and achieve therapeutic breathing patterns. By mimicking or enhancing established slow-breathing techniques, the device supports both clinical interventions and wellness practices.

Furthermore, based on growing evidence linking slow, prolonged exhalation to parasympathetic nervous system activation, improved oxygen delivery, and enhanced cardiac efficiency, the device may offer benefit in conditions such as:
- Cardiovascular diseases (hypertension, heart failure, ischemic heart disease).
- Mental health conditions (anxiety, depression, post-traumatic stress disorder, attention disorders).
- Chronic pain syndromes, where controlled breathing reduces sympathetic drive and pain perception.
- Sleep disturbances and insomnia, by facilitating relaxation breathing patterns.

### Mechanism of Action: Respiratory Effects

The device generates a graded expiratory resistance that varies according to device design and user breathing effort. Exhaling against this controlled resistance establishes positive airway pressure, which:
- Stents open collapsible airways in obstructive lung disorders.
- Reduces dynamic hyperinflation and air trapping, thereby improving ventilation efficiency.
- Promotes alveolar recruitment, expanding functional lung capacity.
- Mitigates oxygen desaturation and breathlessness during exertion.

In individuals without chronic airway obstruction, the device provides a mild but sustained expiratory pressure that lengthens the exhalation phase, reduces the overall respiratory rate, and naturally induces deeper inspirations. This regulated breathing cycle produces measurable physiological and psychological benefits, distinguishing the device from spontaneous or inconsistent self-guided breathing maneuvers.

### Effect of Slow Breathing on the Cardiovascular System

Breathing facilitated by the PEP device can reduce respiratory frequency toward the resonant rate of approximately 6 breaths per minute, a rhythm at which heart and lung function are highly synchronized. At this rate:
- Venous return increases, augmenting right ventricular filling and output.
- Cardiorespiratory coupling is enhanced, with respiratory sinus arrhythmia balancing heartbeats between inspiration and expiration.
- The synchronization of respiratory-driven hemodynamic changes with cardiac contractions improves circulatory efficiency.
- Blood pressure reduction is observed through decreased vascular resistance and enhanced baroreceptor sensitivity.

In addition, the device encourages higher tidal volumes and promotes alveolar recruitment, thereby improving oxygenation, reducing dead space ventilation, and enhancing the ventilation-perfusion ratio. These changes optimize pulmonary gas exchange and indirectly support cardiovascular stability.

The modulation of autonomic balance is also notable: slower breathing with positive expiratory pressure increases parasympathetic activity (calming effect, reduced heart rate, lower blood pressure) while inhibiting excessive sympathetic drive (stress-related acceleration of heart rate).

### Effect on Hypertension and Cardiovascular Disorders

Device-guided breathing has been clinically validated as a non-pharmacological intervention for hypertension. By inducing slow, prolonged exhalation, the device replicates and simplifies the mechanisms of more complex biofeedback systems such as the device known under the trade name RESPeRATE^{™} . Unlike electronic systems requiring sensors, belts, or headphones, the PEP device achieves similar hemodynamic benefits through its mechanical regulation of expiratory resistance, making it portable, passive, and user-friendly.

Regular use may therefore assist in:
- Lowering systolic and diastolic blood pressure.
- Reducing cardiac workload in individuals with heart failure.
- Improving vascular compliance and endothelial function.

### Neurological and Psychological Effects of Slow Breathing

Breathing through the device influences central nervous system activity by altering respiratory rhythm and vagal stimulation. Documented outcomes of slow, controlled breathing include:
- Enhanced alpha-wave activity and reduced theta activity on EEG, correlating with decreased anxiety, anger, and depressive symptoms.
- Increased activity in cortical and subcortical regions (prefrontal cortex, thalamus, hypothalamus, periaqueductal gray), as shown in functional imaging studies, suggesting direct influence on mood regulation and autonomic control.
- Significant reductions in perceived stress when slow breathing training is practiced consistently over weeks.
- Improved attention, mindfulness, and emotional regulation, making the device useful for individuals with anxiety, PTSD, or focus-related challenges.
Moreover, because the device imposes a consistent breathing pattern, it eliminates variability often seen with self-directed mindfulness breathing, thus making the practice accessible to a wider population.

### Competition and Differentiation

Several devices exist that aim to promote slow breathing or assist with mindfulness:
- RESPeRATE^{™}: An FDA-cleared electronic device using sensors and auditory tones to entrain slow breathing for hypertension management. It is effective but requires setup, electronics, and user adherence to biofeedback training.
- Breathe with b^{™}: An electronic system paired with a phone app, designed to guide and track breathing. Currently patent-pending and app-dependent.
- The Shift^{™} (Komuso Design): A stainless-steel jewelry tube marketed for mindfulness breathing. While portable, it lacks regulated resistance and therapeutic PEP pressure control.

Embodiments of the present invention distinguish themselves by combining airway stenting, lung expansion, and physiologic slow breathing in a simple, non-electronic, hands-free device. Unlike purely wellness-oriented competitors, it provides clinically relevant positive expiratory pressure (4-15 cm H₂O) that directly contributes to lung recruitment, oxygenation, and cardiovascular benefit-bridging the gap between medical therapy and lifestyle practice.

Additional example embodiments of the invention are described in the following clauses:
**Clause 1.** A method of facilitating pulmonary expansion comprising the use of a portable positive expiratory pressure (PEP) device configured to deliver adjustable expiratory backpressure within a range of approximately 4 cm H₂O to 15 cm H₂O, wherein said expiratory pressure promotes alveolar recruitment, enhances lung expansion, and improves pulmonary gas exchange, the method being applicable to both individuals with normal lung function and
patients with conditions associated with reduced alveolar surface area, including but not limited to atelectasis and acute respiratory distress syndrome (ARDS).

**Clause 2.** A therapeutic system for the prevention and treatment of atelectasis, comprising a portable PEP device that generates controlled positive airway pressure during exhalation, wherein:
- the positive airway pressure supports alveolar stability and lung re-expansion,
- the user performs slow and prolonged expiratory maneuvers facilitated by the device, thereby promoting deep inspiration, and
- the combination of alveolar recruitment and deep breathing reduces the risk of atelectasis formation and supports clinical management of existing atelectasis.

**Clause 3.** A respiratory training device as described in Clause 2, wherein the configuration of the PEP device allows the user to perform a natural deep inhalation followed by a comfortable, sustained exhalation against resistance, thereby offering a more intuitive and patient-compliant alternative to conventional incentive spirometry techniques.

**Clause 4.** A mechanism for breath regulation comprising the PEP device of Clause 3, wherein exhalation against resistance results in:
- a measurable reduction in respiratory rate by at least 20%;
- prolongation of the expiratory phase relative to inspiration; and
- enhanced conscious awareness and regulation of the breathing cycle, thereby improving breath control, mindfulness, stress reduction, and mitigation of subjective breathlessness.

**Clause 5.** A system for improving arterial oxygenation in subjects with pulmonary dysfunction, wherein use of the PEP device enhances alveolar ventilation, optimizes ventilation-perfusion matching, and increases oxygen diffusion efficiency, thereby benefiting individuals with hypoxic disorders including but not limited to chronic obstructive pulmonary disease (COPD), excessive dynamic airway collapse (EDAC), atelectasis, ARDS, and hypoxemic respiratory failure.

**Clause 6.** A method of inducing parasympathetic nervous system activity through controlled respiratory maneuvers comprising exhalation against positive expiratory resistance, wherein the PEP device facilitates:
- a slowed breathing cadence;
- increased vagal tone; and
- physiological relaxation responses, including decreased heart rate and reduced systemic blood pressure.

**Clause 7.** The method of Clause 6, wherein repeated use of the PEP device provides a non-pharmacological intervention to reduce blood pressure, mitigate cardiac workload, and improve cardiovascular homeostasis in patients with hypertension and related cardiovascular conditions.

**Clause 8.** A respiratory training device for strengthening inspiratory and expiratory muscles, wherein the application of expiratory resistance creates a mild, progressive load on thoracic and abdominal musculature, leading to improved muscle endurance and function following repeated use over time.

**Clause 9.** A method for augmenting venous return and cardiac output comprising the use of the PEP device to generate elevated intra-thoracic pressure during exhalation, combined with rapid inspiratory maneuvers, thereby creating biphasic intra-thoracic pressure fluctuations that enhance venous return and improve cardiac function, particularly in patients with heart failure or other circulatory impairments.

**Clause 10.** A therapeutic system for respiratory rehabilitation comprising the PEP device of Clause 13, wherein regulated exhalation and controlled breathing cycles are applied for retraining respiratory patterns in conditions including lung disease, chronic breathlessness, stress-related disorders, pregnancy-related dyspnea, and anxiety.

**Clause 11.** A system of stress management comprising the use of a PEP device that enables controlled exhalation and paced breathing, wherein said system reduces sympathetic arousal, mitigates post-traumatic stress disorder (PTSD) symptoms, and improves attention, focus, and overall cognitive performance.

**Clause 12.** A method of facilitating sleep onset and treating insomnia comprising the use of a PEP device to guide users into a slow, controlled breathing pattern, wherein said pattern mimics clinically validated relaxation and cognitive-behavioral breathing therapies for sleep induction.

**Clause 13.** A portable, lightweight, non-pharmacological, hands-free positive expiratory pressure (PEP) device comprising:
- a rigid, hollow conical body;
- a wide proximal opening configured to be placed in the user's mouth;
- one or more distal orifices of smaller diameter configured to provide variable airflow resistance;
- said orifices being dimensioned such that exhalation through the device produces positive expiratory airway pressure sufficient to slow the respiratory rate and prolong exhalation; and
- said device being operable without external power supply or batteries, thereby offering a mechanical solution for pulmonary expansion, breath regulation, and therapeutic respiratory conditioning.

It is contemplated that the various embodiments and features described above, including variations thereof, may be combined with one another, even if a specific combination of features is not shown in one of the accompanying figures or described in association with one particular embodiment. From the above disclosure of the general principles of the present invention and the preceding detailed description of exemplifying embodiments, those skilled in the art will readily comprehend the various modifications to which this invention is susceptible. Accordingly, this invention is intended to be limited only by the scope of the following claims and equivalents thereof.

## Claims

1. A method of facilitating pulmonary expansion comprising the use of a portable positive expiratory pressure (PEP) device configured to deliver adjustable expiratory backpressure within a range of approximately 4 cm H₂O to 15 cm H₂O, preferably wherein said expiratory pressure promotes alveolar recruitment, enhances lung expansion, improves pulmonary gas exchange, and / or oxygenation, the method being applicable to individuals with normal lung function and / or patients with conditions associated with reduced alveolar surface area, including but not limited to atelectasis, acute respiratory distress syndrome (ARDS) and hypoxic respiratory failure.

2. A therapeutic system for the prevention and treatment of atelectasis, comprising a portable PEP device that generates controlled positive airway pressure during exhalation, wherein:
• the positive airway pressure supports alveolar stability and lung re-expansion,
• the user performs slow and prolonged expiratory maneuvers facilitated by the device, thereby promoting deep inspiration, and
• the combination of alveolar recruitment and deep breathing reduces the risk of atelectasis formation and supports clinical management of existing atelectasis.

3. The therapeutic system of claim 2, wherein the configuration of the PEP device allows the user to perform a natural deep inhalation followed by a comfortable, sustained exhalation against resistance, thereby offering a more intuitive and patient-compliant alternative to conventional incentive spirometry techniques.

4. The therapeutic system of any of claims 2 or 3, wherein exhalation against resistance results in at least one of:
• a measurable reduction in respiratory rate by at least 20%;
• prolongation of the expiratory phase relative to inspiration;
• enhanced conscious awareness and regulation of the breathing cycle, thereby improving breath control, mindfulness, focus, stress reduction, and mitigation of subjective breathlessness, and / or
• whereas this slow, prolonged, and mindful pattern of controlled breathing mimics clinically validated relaxations and cognitive-behavioral breathing therapies such as those used for sleep induction and insomnia management.

5. A method of inducing parasympathetic nervous system activity through controlled respiratory maneuvers comprising exhalation against positive expiratory resistance, wherein a PEP device facilitates:
• a slowed breathing cadence;
• increased vagal tone; and
• physiological relaxation responses, including decreased heart rate and reduced systemic blood pressure.

6. The method of claim 5, wherein repeated use of the PEP device provides a non-pharmacological intervention to reduce blood pressure, mitigate cardiac workload, and improve cardiovascular homeostasis in patients with hypertension and related cardiovascular conditions.

7. A portable positive expiratory pressure (PEP) device configured to deliver adjustable expiratory backpressure.

8. The portable positive expiratory pressure device of claim 7, wherein the adjustable expiratory backpressure is within a range of approximately 4 cm H₂O to 15 cm H₂O, in particular in the range of 4 cm H₂O to 15 cm H₂O.

9. The portable positive expiratory pressure device of any of claims 7 or 8, configured for a hands-free use.

10. The portable positive expiratory pressure device of any of claims 7 to 9, comprising a rigid, hollow conical body.

11. The portable positive expiratory pressure device of any of claims 7 to 10, comprising a, preferably wide, proximal opening configured to be placed in a user's mouth.

12. The portable positive expiratory pressure device of any of claims 7 to 11, comprising one or more distal orifices configured to provide an airflow resistance.

13. The portable positive expiratory pressure device of claim 12, wherein the one or more distal orifices are configured to provide variable airflow resistance.

14. The portable positive expiratory pressure device of any of claims 7 to 13, said orifices being dimensioned such that exhalation through the device produces positive expiratory airway pressure sufficient to slow the respiratory rate and prolong exhalation.

15. The portable positive expiratory pressure device of any of claims 7 to 14, said device being operable without external power supply or batteries.
